# FASCICULE DE BREVET EUROPEEN

(11) **EP 1 511 735 B1**
(45) Date de publication et mention de la délivrance du brevet: **23.06.2010**
(21) Numéro de dépôt: 03752813.0
(22) Date de dépôt: 15.05.2003
(51) Int. Cl.: C07D 233/54, A61K 31/4164, A61P 43/00

(54) **COMPOSES IMIDAZOLIQUES ET LEUR UTILISATION EN TANT QUE LIGANDS RECEPTEURS ALPHA-2 ADRENERGIQUES**
IMIDAZOLVERBINDUNGEN UND IHRE VERWENDUNG ALS ALPHA-2 ADRENOREZEPTORLIGANDEN
IMIDAZOLE COMPOUNDS AND THEIR USE AS LIGANDS OF THE ALPHA-" ADRENORECEPTORS

(30) Priorité: 16.05.2002 FR 0206026
(43) Date de publication de la demande: 09.03.2005
(73) Titulaire: PIERRE FABRE MEDICAMENT, 92100 Boulogne-Billancourt (FR)
(72) Inventeur: VACHER, Bernard, F-81100 Castres (FR); BONNAUD, Bernard, F-81090 Lagarrigue (FR); MARIEN, Marc, F-81100 Castres (FR); PAUWELS, Peter, F-74270 Chessenaz (FR)
(74) Mandataire: Ahner, Francis
(86) Numéro de dépôt international: PCT/FR2003/001476
(87) Numéro de publication internationale: WO 2003/097611

(56) Documents cités:
- WO-A-01/85698

## Description

La présente invention concerne de nouveaux composés imidazoliques. Les dérivés de l'invention interagissent sélectivement avec les récepteurs adrénergiques du type alpha-2 pré- et/ou post-synaptiques (J. Neurochem. 2001, 78, 685-93) au niveau desquels ils se comportent comme des agonistes partiels, des antagonistes ou des agonistes inverses. En tant que tel, les composés de l'invention sont donc potentiellement utiles dans le traitement des pathologies ou des conditions sensibles à une régulation adrénergique contrôlée par les récepteurs alpha-2 adrénergiques. La liste des pathologies considérées comme sensibles à une telle régulation est excessivement longue. Le champ d'application de la présente invention se limite, toutefois, au traitement des maladies neurodégénératives ainsi qu'au traitement de l'évolution de celles-ci (Psychopharmacology 1996, 123(3), 239-49 ; Prog. Neuro-Psychopharmacol. Biol. Psychiatry 1999, 23(7), 1237-46 ; US 5663167 ; FR 2789681 ; WO 9835670 ; WO 9806393 ; WO 9500145 ; WO 9413285 ; WO 9118886), en particulier au traitement de la maladie ou au traitement de l'évolution de la maladie d'Alzheimer (US 5281607 ; FR 2795727 ; WO 9501791 ; WO 9415603).

La maladie d'Alzheimer est la maladie dégénérative progressive la plus répandue dans la population âgée. Il est estimé que plus de 15 millions de personnes en sont atteintes (New Engl. J. Med. 1999, 341(22), 1670-79 ; Drug Benefit Trends 2001, 13/7, 27-40). Les inhibiteurs d'acétylcholinestérase (e.g., tacrine, donépézil, rivastigmine et galantamine) constituent, à l'heure actuelle, le seul traitement symptomatique de cette maladie. Les bénéfices thérapeutiques obtenus sont, cependant, tout au plus modestes (Drugs 2001, 61/1, 41-52). Les stratégies thérapeutiques efficaces contre la maladie d'Alzheimer étant limitées (Curr. Opin. Invest. Drugs 2001, 2(5), 654-56), la découverte de nouveaux traitements mettant en oeuvre des molécules dotées d'un mécanisme d'action différent de celui des molécules actuellement disponibles en clinique et capables de traiter ou de retarder l'évolution de la maladie est donc fortement souhaitable.

Il a été montré, in vitro et chez l'animal, qu'une substance activant le système noradrénergique peut d'une part s'opposer à la progression de la dégénérescence des neurones (J. Neurophysiol. 1998, 79(6), 2941-63 ; Pharmacol. Biochem. Behav. 1997, 56(4), 649-55 ; J. Cereb. Blood Flow Metabolism 1990, 10(6), 885-94) et, d'autre part, stimuler la croissance neuronale (J. Comp. Neurol. 1974, 155(1), 15-42 ; Neuroscience 1979, 4(11), 1569-82 ; Neuroreport 1991, 2, 528-8). Il s'ensuit que des composés possédant des propriétés agonistes partielles, antagonistes ou agonistes inverses au niveau des récepteurs alpha-2 adrénergiques, en particulier au niveau des récepteurs alpha-2 pré-synaptiques, peuvent être utiles dans le traitement des maladies neurodégénératives. Compte tenu du potentiel thérapeutique des composés dotés de propriétés agoniste partiel, antagoniste ou agoniste inverse pour les récepteurs alpha-2 adrénergiques la découverte de structures nouvelles dotés de telles propriétés est fortement souhaitable. A ce titre, la demanderesse a découvert que de nouveaux dérivés imidazoliques interagissent sélectivement avec les récepteurs adrénergiques du sous type alpha-2 au niveau desquels ils se comportent comme des agonistes partiels, antagonistes ou des agonistes inverses.

De nombreux antagonistes et/ou agonistes partiels pré- et/ou post-synaptiques des récepteurs alpha-2 adrénergiques sont connus et décrits dans la littérature. Bien que les composés en question appartiennent à des classes chimiques différentes (Idrugs 2001, 4(6), 662-76), certains comportent dans leur structure chimique un motif commun du type 1H-imidazole. Parmi ces derniers, on peut citer, à titre d'exemples, des composés du type :
. 4-(1-indanylalkyl)- (WO 1051472) ;
. 4-(benzothiényl)- (WO 9951593) ;
. dihydro-indole- (FR 2735776) ;
. dihydro-indényl- (EP 247764) ;
. 4-(5-fluoro-2,3-dihydro-1H-indèn-2-yl)- (WO 9500492) ;
. 4(5)-(2-éthyl-2,3-dihydro-2-silalndèn-2-yl)- (Eur. J. Med. Chem. 1996, 31(9), 725-9 ;
. thiéno[3,4-c]pyrroles (EP 599697) ;
. 4-(2-aryl- et -cycloalkyl-3,3,3-trifluoropropyl)- (EP 486385) ;
. 4-substituté-imidazole (J. Med. Chem. 1992, 35(4), 750-5) ;
. 4(5)-(2,2-diphényléthyl)- (Eur. J. Med. Chem. 1990, 25(7), 557-68) ;
. dérivés d'imidazoles (GB 2225782 ; EP 183492 et WO 9313074) ;
. 4-(5-fluoro-2,3-dihydro-1H-indèn-2-yl)- (WO 9500492).

Parmi les composés cités ci-dessus, certains ne présentent que des différences structurales relativement minimes. L'état de la technique le plus proche est représenté par des composés du type indanylimidazoles polycycliques, revendiqués dans le brevet WO 0185698, répondant à la formule a suivante : 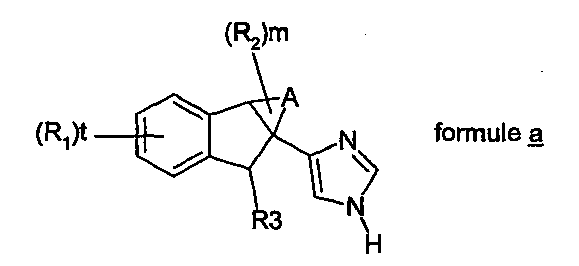 dans laquelle, entre autres :
- A peut former, avec les deux atomes de carbone par lesquels il est attaché, un mono-cycle carboné a 3 chaînons ;
- m peut être 0 ou 1;
- R2 peut être un groupe (C1-6)alcoyle
- t peut être 0 ou 1 ;
- t est 1 et R1 peut être un halogène ou un groupe (C1-6)alcoyloxy ;
- R3 peut être un hydrogène, OH, =O, (C1-6)alcoyle ou (C1-6)alcoyloxy.

Les composés représentés ci-dessus et les composés de la présente invention se différencient donc par la nature du substituant en position 4 du groupe imidazole, en particulier par la présence dans les composés de l'invention d'un motif 6-spiro-cyclopropane. Nombre de structures faisant intervenir un groupe 1H-imidazole substitué en position 4 sont déjà connus pour leurs propriétés alpha-2 adrénergiques (vide supra). Cependant, de manière surprenante, il apparaît que le substituant 6-spiro-1'-cyclopropane-1a,6-dihydro-1H-cyclopropa[a]indèn-6a-yl confère aux composés de l'invention un profil pharmacologique tout à fait singulier.

En effet, nous montrons, in vitro :
- une affinité des composés de l'invention vis-à-vis du sous-type alpha-2A humain.
- des propriétés antagonistes ou agonistes inverses des composés de l'invention au niveau des récepteurs alpha-2A.

De plus, nous montrons, in vivo, que les produits de l'invention sont capables de s'opposer à l'effet de la scopolamine dans un test de déficit mnésique. Ce test est considéré comme un modèle animal représentatif des troubles de la mémoire qui se manifestent au cours de la maladie d'Alzheimer (Psychopharmacology 1992, 106, 26-30 ; Eur. J. Clin. Invest. 1998, 28, 944-9 ; Exp. Neurol. 2000, 163, 495-529). Les composés de l'invention, dotés d'un tel profil d'activité, sont donc potentiellement utiles pour le traitement des maladies ou des troubles sensibles à l'action des agonistes partiels, des antagonistes ou des agonistes inverses des récepteurs alpha-2 adrénergiques, en particulier pour le traitement des maladies neurodégénératives pour lesquelles il existe un besoin thérapeutique important.

Enfin, le procédé de préparation des composés de l'invention est différent de celui des composés revendiqués dans le brevet WO 0185698.

Plus spécifiquement, la présente invention a pour objet les nouveaux dérivés 4-(6-spiro-1'-cyclopropane-1a,6-dihydro-1H-cyclopropa-[a]indèn-6a-yl)-1H-imidazole qui, sous forme de base, répondent à la formule générale (1) : 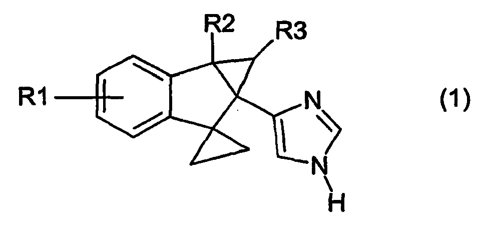 dans laquelle :
- R1 représente un atome d'hydrogène, un atome de fluor ou un groupe méthoxyle (OCH₃), le substituant R1 sur le carbocycle aromatique pouvant occuper la position 2, 3, 4 ou 5 ;
- R2 représente un atome d'hydrogène ou un groupe méthyle ;
- R3 représente un atome d'hydrogène, un groupe méthyle ou un groupe éthyle ;
   leurs sels d'addition et éventuellement les hydrates des sels d'addition avec les acides minéraux ou les acides organiques pharmaceutiquement acceptables ainsi que leurs formes tautomères, les énantiomères et les mélanges d'énantiomères et les stéréoisomères purs ou en mélanges racémique ou non.

Dans un mode particulier de réalisation de l'invention, les composés de formule (1) dans laquelle :
- R1 et R2 ont la même signification que précédemment ;
- R3 représente un groupe méthyle ou un groupe éthyle ;
   les stéréoisomères préférés des produits de l'invention sont ceux dans lesquels les substituants R3 et 1H-imidazole occupent soit des positions anti-périplanaires ; soit des positions syn-périplanaires par rapport au plan défini par le noyau cyclopropanique.

Par anti-périplanaire les inventeurs entendent les configurations relatives des molécules (1) pour lesquelles les substituants R3 et 1H-imldazole sont situés de part et d'autre du plan défini par le noyau cyclopropanique. Par syn-périplanaire les inventeurs entendent les configurations relatives des molécules (1) pour lesquelles les substituants R3 et 1H-imidazole sont situés du même coté du plan défini par le noyau cyclopropanique.

Les composés de formule générale (1) peuvent exister sous plusieurs formes tautomères. De telles formes tautomères, quoique non explicitement rapportées dans la présente demande pour simplifier la représentation graphique des formules développées, sont néanmoins incluses dans le champ d'application de l'invention. Les composés de l'invention comportent plusieurs atomes de carbone asymétriques dans leur structure. De ce fait, ils existent sous la forme d'énantiomères et de diastéréoisomères. L'invention concerne aussi bien chaque stéréoisomère pur, c'est à dire associé avec moins de 5% d'un autre stéréoisomère ou d'un mélange d'autres stéréoisomères, que les mélanges d'un ou plusieurs stéréoisomères en toutes proportions. Les composés de l'invention peuvent donc intervenir en tant que stéréoisomères purs ou mélanges racémiques ou non-racémiques de stéréoisomères.

L'invention s'étend enfin au procédé de préparation des dérivés de formule générale (1).

Les dérivés de formule générale (1) peuvent être obtenus par le procédé décrit dans le schéma réactionnel ci-après.

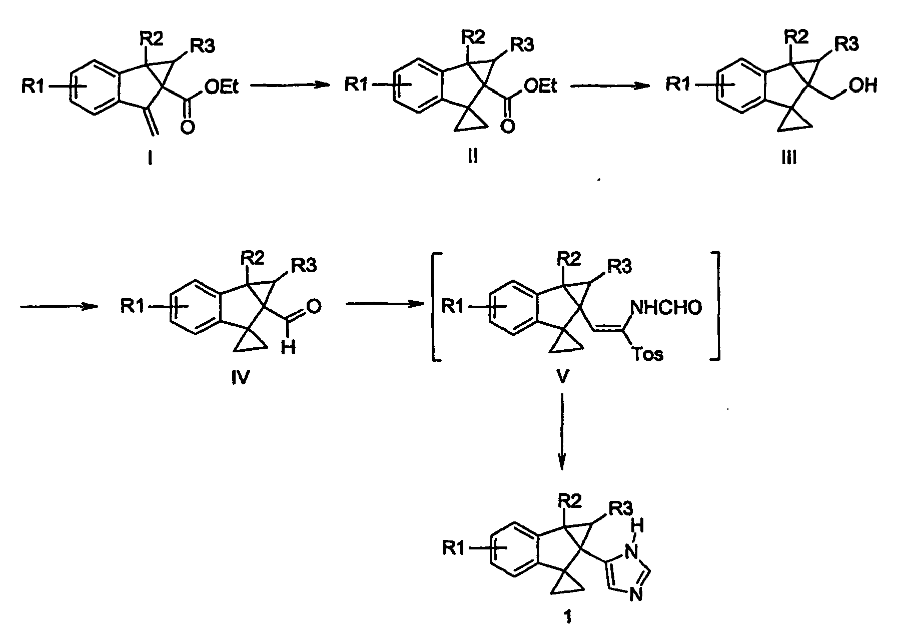

La préparation des composés de l'invention utilise comme matière première les dérivés de formule (I) convenablement substitués dont la méthode de synthèse est décrite dans la demande de brevet français N° 0201839. Une réaction de cyclopropanation de la double liaison, effectuée par une technique analogue à celle rapportée dans Angew. Chem. Int. Ed. 2000, 39(24), 4539-42, conduit au dérivé spiro de formule (II). La fonction ester du composé de formule (II) est ensuite réduite en l'alcool de formule (III) au moyen de borohydrure de lithium dans le tétrahydrofurane selon une méthode classique de la chimie organique. L'alcool primaire (III), est oxydé en l'aldéhyde de formule (IV) au moyen du complexe pyridine trioxyde de soufre. L'aldéhyde (IV) est converti en l'imidazole attendu de formule (1) soit en une étape selon la méthode décrite dans Heterocycles 1994, 39(1), 139-53 ; soit par l'intermédiaire de la tosyl-formylamine de formule (V) selon la méthode rapportée dans Recl. Trav. Chim. Pays-Bas 1979, 98(5), 258-62.

L'invention a aussi pour objet les compositions pharmaceutiques contenant à titre de principe actif au moins un des dérivés de formule générale (1) ou un de ses sels ou hydrates de ses sels en combinaison avec un ou plusieurs support inertes ou autres véhicules pharmaceutiquement acceptables.

Les compositions pharmaceutiques selon l'invention peuvent être, à titre d'exemple, des compositions administrables par voie orale, nasale, sublingual, rectale ou parentérale. A titre d'exemple de compositions administrables par voie orale on peut citer les comprimés, les gélules, les granules, les poudres et les solutions ou suspensions orales.

Les formulations appropriées pour la forme d'administration choisie sont connues et décrites, par exemple dans : Remington, The Science and Practice of Pharmacy, 19ème édition, 1995, Mack Publishing Company.

La dose efficace d'un composé de l'invention varie en fonction de nombreux paramètres tels que, par exemple, la voie d'administration choisie, le poids, l'âge, le sexe, l'état d'avancement de la pathologie à traiter et la sensibilité de l'individu à traiter. En conséquence, la posologie optimale devra être déterminée, en fonction des paramètres jugés pertinents, par le spécialiste en la matière. Bien que les doses efficaces d'un composé de l'invention puissent varier dans de larges proportions, les doses journalières pourraient s'échelonner entre 0,01 mg et 100 mg par Kg de poids corporel de l'individu à traiter. Une dose journalière d'un composé de l'invention comprise entre 0,10 mg et 50 mg par Kg de poids corporel de l'individu à traiter étant, toutefois, préférée.

Les compositions pharmaceutiques selon l'invention sont utiles dans le traitement des maladies neurodégénératives.

### Exemples

Les exemples suivants illustrent l'invention, mais ne la limitent en aucune façon.

Dans les exemples et les exemples de référence ci-après :
(i) l'avancement des réactions est suivi par chromatographie sur couche mince (CCM) et par conséquent les temps de réaction ne sont mentionnés qu'à titre indicatif ;
(ii) des formes cristallines différentes peuvent donner des points de fusion différents, les points de fusion rapportés dans la présente demande sont ceux des produits préparés selon la méthode décrite et ne sont pas corrigés ;
(iii) la structure des produits obtenus selon l'invention est confirmée par les spectres de résonance magnétique nucléaire (RMN), infrarouge (IR) et l'analyse centésimale, la pureté des produits finaux est vérifiée par CCM, la pureté énantiomérique des intermédiaires réactionnels et des produits finaux est déterminée par HPLC sur phase chirale ;
(iv) les spectres RMN sont enregistrés dans le solvant indiqué. Les déplacements chimiques (δ) sont exprimés en partie par million (ppm) par rapport au tétraméthylsilane. La multiplicité des signaux est indiquée par : s, singulet ; d, doublet ; t, triplet ; q, quadruplet ; m, multiplet ; I, large ;
(v) les différents symboles des unités ont leur signification habituelle : µg (microgramme) ; mg (milligramme) ; g (gramme) ; ml (millilitre) ; mV (millivolt) ; °C (degré Celsius) ; mmole (millimole) ; nmole (nanomole) ; cm (centimètre) ; nm (nanomètre) ; min (minute) ; ms (milliseconde), Hz (hertz) ; [α] (pouvoir rotatoire spécifique mesuré à 589 nm, 25°C et à la concentration c, dans la présente invention la dimension deg.cm².g⁻¹ est toujours sous entendue) ; les pressions sont données en millibars (mb) ;
(vi) les abréviations ont la signification suivante : F (point de fusion) ; Eb (point d'ébullition) ; AUC (aire sous la courbe) ;
(vii) par "température ambiante" on entend une température comprise entre 20°C et 25°C.

### Exemple 1 : (6-spiro-1'-cyclopropane-1a,6-dihydro-1H-cyclopropa-[a]indèn-6a-yl)-carboxylate d'éthyle (II-1)

Dans la solution de 24,5 g (0,124 mole) de 2,4,6-trichlorophénol et 300 ml de dichlorométhane sous agitation et sous azote à - 40°C, on ajoute goutte à goutte 113 ml (0,124 mole) d'une solution toluènique de ZnEt₂ (1,1M). Après 15 minutes d'agitation à - 40°C on ajoute 10 ml (0,124 mole) de diiodométhane et maintient 15 minutes sous agitation avant d'ajouter le 6-méthylène-1a,6-dihydro-1H-cyclopropa[a]Indèn-6a-carboxylate d'éthyle (I-1), 13,22 g (0,062 mole). La suspension obtenue est maintenue sous agitation à température ambiante pendant la nuit. Après rajout de dichlorométhane jusqu'à dissolution complète, la solution est lavée 2 fois par HCl 1N puis Na₂SO₃ puis NaOH 0,5N (2 fois) puis à l'eau salée. La phase organique est séchée sur MgSO₄, filtrée et le solvant est éliminé sous vide. L'huile obtenue est purifiée par chromatographie sur gel de silice en utilisant le cyclohexane à 2 % d'acétate d'éthyle comme éluant.
Rendement : 89,8 %
C₁₅H₁₆O₂ : 228,29
¹H RMN (CDCl₃) : 0,88 (m, 1H); 0,92 (m, 2H); 1,23 (t, 3H); 1,29 (m, 1H); 1,81 (dd, 1H); 2,35 (m, 1H); 3,17 (dd, 1H); 4,10 (q, 2H); 6,56 (m, 1H); 7,07 (m, 2H); 7,25 (m, 1H).
¹³C RMN (CDCl₃) : 14,15; 14,47; 17,16; 27,47; 29,21; 34,09; 34,16; 60,22; 118,97; 122,76; 125,40; 126,43; 142,46; 147,45; 171,84.

### Exemple 2 : (6-spiro-1'-cyclopropane-1a,6-dihydro-1H-cyclopropa-[a]indèn-6a-yl)-méthanol (III-1)

La suspension de 13 g (0,234 mole) de KBH₄, 10,5 g (0,239 mole) de LiCl et 100 ml de THF anhydre est maintenue sous agitation à température ambiante pendant 1 heure. A cette suspension on ajoute goutte à goutte la solution de 12,71 g, (0,056 mole) de (6-spiro-1'-Cyclopropane-1a,6-dihydro-1H-cyclopropa[a]indèn-6a-yl)-carboxylate d'éthyle (II-1) dans 70 ml de THF anhydre puis on porte au reflux sous agitation pendant 4 heures. La suspension est concentrée sous vide et le résidu est traité à l'eau. Le produit est extrait 2 fois par l'acétate d'éthyle. La phase organique est lavée à l'eau salée, séchée sur MgSO₄, filtrée et concentrée sous vide. L'huile brute est purifiée par chromatographie sur silice, en utilisant le cyclohexane à 20 % d'acétate d'éthyle comme éluant.
Rendement : 85 %
C₁₃H₁₄O : 186,25
¹H RMN (CDCl₃) : 0,64 (t, 1H); 0,96 (m, 2H); 1,17 (m, 2H); 1,25 (t, 1H, échangeable avec D₂O); 1,54 (m, 1H); 2,49 (q, 1H); 3,56 (dd, 1H, (d, après échange avec D₂O)); 3,74 (dd, 1H, (d, après échange avec D₂O)); 6,58 (m, 1H); 7,06 (m, 2H); 7,25 (m, 1H).

### Exemple 3 : (6-spiro-1'-cyclopropane-1a,6-dihydro-1H-cyclopropa-[a]indèn-6a-yl)-carboxaldéhyde (IV-1)

Dans la solution de 1,2 g (6,44 mmoles) de (6-spiro-1-Cyclopropane-1a,6-dihydro-1H-cyclopropa[a]inden-6a-yl)-méthanol (III-1) et 6 ml de DMSO anhydre, on ajoute 2,7 ml (19,4 mmoles) de triéthylamine. Le mélange obtenu est mis sous agitation sur un bain d'eau glacée et l'on ajoute par fractions 3,1 g (19,4 mmoles) de complexe pyridine-SO₃. Après 4 heures d'agitation à température ambiante, la solution est versée dans l'eau glacée. Le produit est extrait deux fois par l'acétate d'éthyle. La phase organique est lavée par une solution aqueuse d'acide citrique puis à l'eau salée. Après séchage, sur MgSO₄ et filtration, le solvant est éliminé sous pression réduite. L'huile résiduelle obtenue est utilisée sans autre purification dans l'étape suivante.
C₁₃H₁₂O : 184,23
¹H RMN (CDCl₃) : 0,97-1,03 (m, 2H); 1,15 (t, 1H); 1,23 (m, 1H); 2,01 (dd, 1H); 2,37 (m, 1H); 3,15 (dd, 1H); 6,62 (d, 1H); 7,12 (m, 2H); 7,26 (t, 1H); 9,26 (s, 1H).

### Exemple 4: 4-(6-spiro-1'-Cyclopropane-1a,6-dihydro-1H-cyclopropa[a]indèn-6a-yl)-1H-imidazole (1-1)

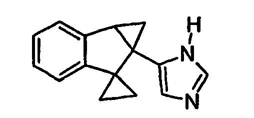

Dans la suspension de 1,18 g (6,4 mmoles) de (6-spiro-l'-Cyclopropane-1a,6-dihydro-1H-cyclopropa[a]indèn-6a-yl)-carboxaldéhyde (IV-1), 1,25 g (6,4 mmoles) d'isocyanure de para-tolylsulfonylméthyle et 15 ml d'éthanol absolu sous agitation à température ambiante, on ajoute 40 mg de cyanure de sodium. Après 1 heure d'agitation à température ambiante la majorité de l'éthanol est éliminé sous pression réduite. On ajoute au résidu 20 ml d'une solution méthanolique d'ammoniac (4N) et maintient la solution obtenue à 90°C pendant 16 heures. Après retour à température ambiante, la solution brune obtenue est amenée à sec sous pression réduite. Le résidu est repris par de l'acétate d'éthyle et les insolubles sont filtrés. Les eaux mères sont extraites 2 fois par l'acide chlorhydrique 1N. Les phases aqueuses acides sont lavées par l'éther puis alcalinisées. Le produit est extrait 2 fois par l'acétate d'éthyle. Les phases organiques sont lavées à l'eau salée, séchées sur MgSO₄, filtrées et le solvant est éliminé sous pression réduite. Le résidu est purifié par chromatographie sur silice en utilisant le chloroforme à 3 % de méthanol comme éluant.
Rendement : 28,8 %
C₁₅H₁₄N₂ : 222,28
Fumarate du composé du titre, F : 218-220° C
Analyse élémentaire, C₁₉H₁₈N₂O₄ : 338,36
Calculé : C 67,45 %, H 5,36 %, N 8,28 %
Trouvé : C67,17 %, H 5,36 %, N 8,15 %
¹H RMN (DMSOd₆) : 0,61 (t, 1H); 0,79 (m, 2H); 0,94 (m, 1H); 1,09 (m, 1H); 1,46 (dd, 1H); 2,71 (dd, 1H); 6,62 (s, 2H); 6,68 (m, 1H); 6,90 (s, 1H); 7,06 (m, 2H); 7,28 (m, 1H); 7,63 (s, 1H).

Le dédoublement des composés de formule (1-1) est effectué par chromatographie liquide sur un support CHIRALCEL OD.

### Exemple 5 : (+)-4-(6-spiro-1-cyclopropane-1a,6-dihydro-1H-cyclopropa[a]indèn-6a-yl)-1H-imidazole (+)-(1-1)

Fumarate du composé du titre, F : 168-170° C
[α]²⁵_{D} = + 50,5° (c = 0,334, CH₃OH)
Analyse élémentaire, C₁₉H₁₈N₂O₄ 338,36
Calculé : C 67,45 %, H 5,36 %, N 8,28 %
Trouvé : C 67,24 %, H 5,39 %, N 8,12 %

### Exemple 6 : (-)-4-(6-spiro-1'-cyclopropane-1a,6-dihydro-1H-cyclopropa[a]indén-6a-yl)-1H-imidazole (-)-(1-1)

Fumarate du composé du titre, F : 170-172° C
[α]²⁵_{D} = - 47,7° (c = 0,295, CH₃OH)
Analyse élémentaire, C₁₉H₁₈N₂O₄ : 338,36
Calculé : C 67,45 %, H 5,36 %, N 8,28 %
Trouvé : C 67,23 %, H 5,36 %, N 8,16 %

### Exemple 7: 4-(1-exo-méthyl-6-spiro-1'-cyclopropane-1a,6-dihydro-1H-cyclopropa[a]indèn-6a-yl)-1H-imidazole (1-2)

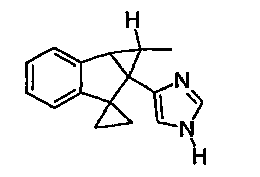

En utilisant comme produit de départ le 1-exo-méthyl-6-méthylène-1a,6-dihydro-1H-cyclopropa[a]indèn-6a-carboxylate d'éthyle (I-2) et en procédant comme décrit dans les exemples 1-4, on obtient le composé du titre.
Fumarate du composé du titre, F : 203-205°C
Analyse élémentaire, C₂₀H₂₀N₂O₄ : 352,39
Calculé : C 68,17 %, H 5,72 %, N 7,95 %
Trouvé : C 68,69 %, H 5,90 %, N 8,07 %
¹H RMN (DMSOd₆) : 0,57 (m, 1H); 0,65 (m, 1H); 0,88 (s, 3H); 0,92 (m, 1H); 1,28 (m, 1H); 2,54 (d, 1H, J = 1,6 Hz); 6,61 (s, 2H); 6,63 (m, 1H); 6,68 (s, 1H); 7,04 (m, 2H); 7,28 (m, 1H); 7,66 (s, 1H).

### Exemple 8: 4-(1-endo-éthyl-6-spiro-1'-cyclopropane-1a,6-dihydro-1H-cyclopropa[a]indèn-6a-yl)-1H-imidazole (1-3)

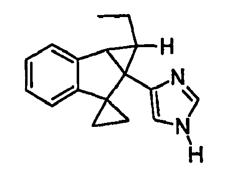

En utilisant comme produit de départ le (1-endo-éthyl-6-méthylène-1a,6-dihydro-1H-cyclopropa[a]indèn-6a-carboxylate d'éthyle (I-3), lui-même obtenu à partir de l'acide (Z)-2-(1-butenyl)-benzoïque (RN 129780-54-7) et en procédant comme décrit dans les exemples 1-4, on obtient le composé du titre.
Fumarate du composé du titre, F : 179-181 °C
Analyse élémentaire, C₂₁H₂₂N₂O₄ : 366,42
Calculé : C 68,84 %, H 6,05 %, N 7,64 %
Trouvé : C 68,36 %, H 5,99 %, N 7,63 %
¹H RMN (D₂O) : 0,62 (m, 1H); 0,86 (t, 3H); 0,95 (m, 2H); 1,14 (m, 1H); 1,20 (m, 1H); 1,38 (m, 1H); 1,74 (m, 1H); 3,03 (d, 1H, J = 8,8 Hz); 6,66 (s, 2H); 6,75 (m, 1H); 7,21 (m, 2H); 7,31 (s, 1H); 7,39 (m, 1H); 8,56 (s, 1H).

### Exemple 9 : 4-(1a-méthyl-6-spiro-1'-cyclopropane-1a,6-dihydro-1H-cyclopropa[a]indèn-6a-yl)-1H-imidazole (1-4)

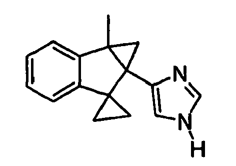

En utilisant comme produit de départ le 1la-méthyl-6-méthylène-1a,6-dihydro-1H-cydopropa[a]Indèn-6a-yl)-carboxylate d'éthyle (I-4), lui-même obtenu à partir de l'acide 2-isopropényl-benzoique (RN 3609-46-9) et en procédant comme dans les exemples 1-4, on obtient le composé du titre.

### Exemple 10 : 4-(4-Fluoro-6-spiro-1'-cyclopropane-1a,6-dihydro-1H-cyclopropa[a]indèn-6a-yl)-1H-imidazole (1-5)

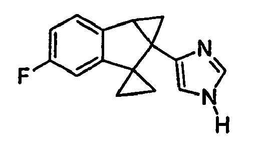

En utilisant comme produit de départ le (4-fluoro-6-méthylène-1a,6-dihydro-1H-cyclopropa[a]indèn-6a-yl)-carboxylate d'éthyle (I-5) et en procédant comme dans les exemples 1-4, on obtient le composé du titre.
Fumarate du composé du titre, F : 214-216°C
Analyse élémentaire, C₁₉H₁₇N₂FO₄ : 356,35
Calculé : C 64,04 %, H 4,81 %, N 7,86 %
Trouvé : C 63,87 %, H 4,88 %, N 7,81 %
¹H RMN (DMSOd₆) : 0,60 (t, 1H); 0,83 (m, 2H); 0,98 (m, 1H); 1,09 (m, 1H); 1,42 (dd, 1H); 2,68 (dd, 1H); 6,56 (d, 1H); 6,66 (s, 2H); 6,83 (m, 1H); 6,88 (s, 1H); 7,27 (m, 1H); 7,59 (m, 1H).

Les composés de formule (1) ainsi que leurs sels thérapeutiquement acceptables présentent des propriétés pharmacologiques intéressantes.

Les résultats des essais sont regroupés dans le tableau suivant :

| **Composé** | **Affinité** (pKi) | **Activité Intrinsèque** | **Déficit mnésique à la scopolamine** |
|---|---|---|---|
| | Alpha-2A | % stimulé | amplitude de l'effet % (dose, mg/kg i.p.) |
| 1-1 | 9,5 | +14 | +122 (2,5) |
| (-)-adrénaline | - | +100 | - |
| Donépézil | - | - | +67 (0,16) |

### Liaisons aux récepteurs alpha-2 adrénergiques.

Les membranes des cellules C6 exprimant de façon permanente le récepteur humain alpha-2A sont préparés dans du Tris-HCl (pH = 7,6). Les essais de liaison sont effectués avec 2 nM [³H]RX 821002. Le milieu d'incubation est composé de 0,4 ml de membranes cellulaires (10 µg de protéines), 0,05 ml de radioligand et 0,05 ml de produit à tester ou de phéntolamine (10 µM) pour déterminer la liaison non spécifique. La réaction est arrêtée après 30 minutes d'incubation à 25°C en ajoutant 3 ml de Tris-HCl, 50 mM (pH = 7,6), froid, suivie d'une filtration sur filtres Whatman, GF/B à l'aide d'un Brandel. Les valeurs du Ki sont calculée selon l'équation Ki = IC₅₀/(1 + C/Kd) où C est la concentration et Kd la constante de dissociation, pKi = - logKi. Dans ces conditions, il apparaît que les composés de l'invention possèdent une forte affinité pour les récepteurs du sous type alpha-2A adrénergiques humains.

### Mesure de l'activation des récepteurs alpha-2 adrénergiques.

Les réponses en GTPγS sont effectuées sur des préparations membranaires dans l'HEPES 20 mM (pH = 7,4) avec 30 µM de GDP, 100 mM de NaCl, 3 mM de MgCl₂ et 0,2 mM d'acide ascorbique. La stimulation maximale du GTPγS est déterminée en présence de 10 mM de (-)-adrénaline et calculée versus la réponse GTPγS basale. Les résultats sont exprimés versus soit l'adrénaline soit du RX 811059. Dans ces conditions, les composés de l'invention se distinguent de la plupart des composés de l'art antérieur en ce qu'ils se comportent plutôt comme des agonistes inverses au niveau des récepteurs adrénergiques alpha-2A humains (cf. tableau ci-dessus).

### Test du déficit mnésique induit par la scopolamine.

La scopolamine possède des propriétés amnésiantes chez l'animal et l'homme. Ainsi, son administration chez l'homme sain provoque certains symptômes proches de ce qui est observé dans la maladie d'Alzheimer. Le déficit mnésique induit par la scopolamine est donc utilisé comme modèle pharmacologique expérimental des troubles de mémoire qui se manifestent au cours de cette pathologie. La scopolamine réduit la capacité d'acquisition, de mémorisation et de rappel dans un test d'évitement passif chez le rat. Il s'agit de mesurer la réticence, après apprentissage, qu'éprouve un animal à entrer dans un compartiment sombre où il reçoit un choc électrique de faible intensité. L'administration de scopolamine supprime cette réticence, et les composés étudiés s'opposent à l'effet de la scopolamine. Le protocole expérimental utilisé est décrit dans Psychopharmacol. 1992, 106, 26-30.

Les composés de l'invention montrent une activité importante (cf. tableau ci-dessus). L'amplitude de l'effet obtenu avec les composés de l'invention est supérieur à celui, par exemple, du donézépil, inhibiteur d'acétylcholinestérase utilisé en clinique pour le traitement de la maladie d'Alzheimer (Chem. Rec. 2001, 1(1), 63-73). Les composés de l'invention sont donc capables de s'opposer efficacement au déficit mnésique induit par la scopolamine.

Les résultats des essais montrent donc que les composés de formule (1) :
- possèdent une forte affinité pour les récepteurs adrénergiques du sous type alpha-2A humains ;
- se comportent, généralement, comme des agonistes partiels ou antagonistes ou des agonistes inverses au niveau des récepteurs adrénergiques alpha-2A humains ;
- sont actifs, in vivo, dans un modèle animal considéré comme représentatif des troubles de la mémoire qui se manifestent au cours de la maladie d'Alzheimer.

De ce fait, les composés de l'invention ainsi que leurs sels thérapeutiquement acceptables sont potentiellement utiles comme médicaments, en particulier dans le traitement de certaines pathologies neurodégénératives progressives telle que, par exemple, la maladie d'Alzheimer.

L'administration des composés de l'invention peut être réalisée par voie orale, nasale, sublinguale, rectale ou parentérale. A titre d'exemples de formulation non limitatifs, nous donnons ci-après, une préparation des composés de l'invention. Les ingrédients ainsi que d'autres, thérapeutiquement acceptables, peuvent être introduits en d'autres proportions sans modifier la portée de l'invention. Les termes 'ingrédient actif utilisés dans l'exemple de formulation ci-après font référence à un composé de formule (1) ou un sel d'addition ou éventuellement un hydrate d'un sel d'addition du composé de formule (1) avec un acide minéral ou un acide organique pharmaceutiquement acceptable.

### Exemple de composition pharmaceutique

Formule de préparation pour 1000 comprimés contenant chacun 10 mg de l'ingrédient actif constitué par au moins un composé imidazolique selon l'invention :

| | |
|---|---|
| Ingrédient actif | 10 g |
| Lactose | 100 g |
| Amidon de blé | 10 g |
| Stéarate de magnésium | 3 g |
| Talc | 3 g |

## Revendications

1. Composés de formule générale (1): 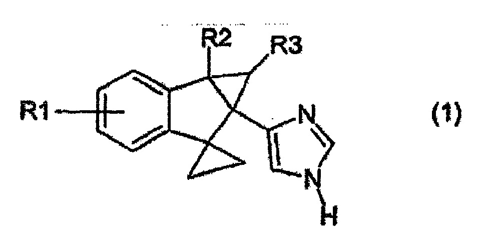 dans laquelle:
- R1 représente un atome d'hydrogène, un atome de fluor ou un groupe méthoxyle (OCH3), le substituant R1 sur le carbocycle aromatique pouvant occuper la position 2, 3, 4 ou 5 ;
- R2 représente un atome d'hydrogène ou un groupe méthyle
- R3 représente un atome d'hydrogène, un groupe méthyle ou un groupe éthyle ;
leurs sels d'addition et éventuellement les hydrates des sels d'addition avec les acides minéraux ou les acides organiques pharmaceutiquement acceptables ainsi que leurs formes tautomères, les énantiomères et les mélanges d'énantiomères et les stéréoisomères purs ou en mélange racémique ou non.

2. Composés selon la revendication 1, **caractérisé en ce qu'**ils sont choisis parmi les composés suivants :
4-(6-spiro-1'-Cyclopropane-1a,6-dihydro-1H-cyclopropa[a]indèn-6a-yl)-1H-imidazole ;
4-(2-fluoro-6-spiro-1'-cyclopropane-1a,6-dihydro-1H-cyclopropa[a]indèn-6a-yl)-1H-imidazole;
4-(3-fluoro-6-spiro-1'-cyclopropane-1a,6-dihydro-1H-cyclopropa[a]indèn-6a-yl)-1H-imidazole;
4-(4-fluoro-6-spiro-1'-cyclopropane-1a,6-dihydro-1H-cyclopropa[a]indèn-6a-yl)-1H-imidazole;
4-(5-fluoro-6-spiro-l'-cyclopropane-1a,6-dihydro-1H-cyclopropa[a]indèn-6a-yl)-1H-imidazole ;
4-(1-exo-méthyl-6-spiro-1'-cyclopropane-1a,6-dihydro-1H-cyclopropa[a]indèn-6a-yl)-1H-imidazole ;
4-(1-exo-méthyl-2-fluoro-6-spiro-1'-cyclopropane-1a,6-dihydro-1H-cyclopropa[a]indèn-6a-yl)-1H-imidazole;
4-(1-exo-méthyl-3-fluoro-6-spiro-1'-cyclopropane-1a,6-dihydro-1H-cyclopropa[a]indèn-6a-yl)-1H-imidazole;
4-(1-exo-méthyl-4-fluoro-6-spiro-1'-cyclopropane-1a,6-dihydro-1H-cyclopropa[a]indèn-6a-yl)-1H-imidazole ;
4-(1-exo-méthyl-5-fluoro-6-spiro-1'-cyclopropane-1a,6-dihydro-1H-cyclopropa[a]indèn-6a-yl)-1H-imidazole;
4-(11-endo-éthyl-6-spiro-1'-cyclopropane-1a,6-dihydro-1H-cyclopropa[a]indèn-6a-yl)-1H-imidazole;
4-(1a-méthyl-6-spiro-1'-cyclopropane-1a,6-dihydro-1H-cyclopropa[a]indèn-6a-yl)-1H-imidazole;
leurs sels d'addition et éventuellement les hydrates des sels d'addition avec les acides minéraux ou les acides organiques pharmaceutiquement acceptables ainsi que leurs formes tautomères, les énantiomères et les mélanges d'énantiomères et les stéréoisomères purs ou en mélange racémique ou non.

3. Procédé de préparation des composés de formule (1) selon les revendications 1 et 2, **caractérisé en ce que** l'on fait réagir un intermédiaire de synthèse de formule (I) : 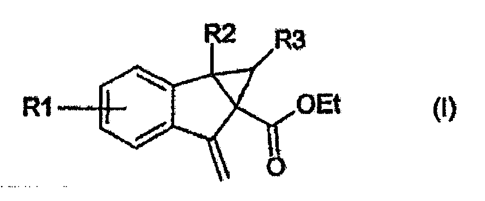 dans laquelle R1, R2 et R3 ont la même signification que dans la formule (1):
avec du diiodométhane en présence de diéthyl zinc et de phénol et que l'on converti la fonction ester du composé formé en un groupe 1H-imidazole pour obtenir les composés de formule (1) dans laquelle R1, R2 et R3 ont la même signification que dans la formule (1), leurs sels d'addition et éventuellement les hydrates des sels d'addition avec les acides minéraux ou les acides organiques pharmaceutiquement acceptables ainsi que leurs formes tautomères, les énantiomères et les mélanges d'énantiomères et les stéréoisomères purs ou en mélange racémique ou non.

4. Composés selon la revendication 1, **caractérisé en ce que** les substituants R3 et 1H-imidazole occupent des positions syn-périplanaires par rapport au plan défini par le noyau cyclopropanique, R1 et R2 ont la même signification que dans la formule (1) et R3 est un groupe méthyle (CH₃) ou un groupe éthyle (CH₂CH₃).

5. Composés selon la revendication 1, **caractérisé en ce que** les substituants R3 et 1H-imidazole occupent des positions anti-périplanaires par rapport au plan défini par le noyau cyclopropanique, R1 et R2 ont la même signification que dans la formule (1) et R3 est un groupe méthyle (CH3) ou un groupe éthyle (CH₂CH₃).

6. Composés de formule générale (1), selon l'une quelconque des revendications 1, 2, 4 ou 5, **caractérisé en ce qu'**il est choisi parmi l'énantiomère lévogyre ou l'énantiomère dextrogyre des composés de formule générale (1).

7. Composés selon l'une des revendications 1, 2, 4, 5 et 6 à titre de médicaments.

8. Compositions pharmaceutiques **caractérisées en ce qu'**elles contiennent comme ingrédient actif au moins un composé selon l'une des revendications 1, 2, 4, 5 et 6, associé à un support pharmaceutique inerte ou autres véhicules pharmaceutiquement acceptables et éventuellement à un autre médicament.

9. Compositions pharmaceutiques selon la revendication 8, utiles dans le traitement de la maladie d'Alzheimer ou utiles dans le traitement de l'évolution de la maladie d'Alzheimer.

10. Compositions pharmaceutiques selon la revendication 8, utiles dans le traitement ou dans le traitement de l'évolution de la maladie de Parkinson.

## Claims

1. Compounds according to general formula (1): 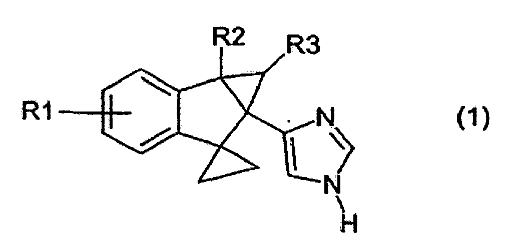 wherein:
- R1 represents a hydrogen atom, a fluorine atom or a methoxyl group (OCH₃), the substituent R1 on the aromatic carbocycle possibly occupying the 2, 3, 4 or 5 position;
- R2 represents a hydrogen atom or a methyl group
- R3 represents a hydrogen atom, a methyl group or an ethyl group;
their addition salts and, if applicable, addition salt hydrates with pharmaceutically acceptable mineral acids or organic acids along with their tautomeric forms, the enantiomers, and the mixtures of enantiomers
and the pure stereoisomers or racemic or non-racemic mixtures.

2. Compounds according to 1, **characterised in that** they are selected from the following compounds:
4-(6-spiro-1'-Cyclopropane-1a,6-dihydro-1H-cyclopropa[a]inden-6a-yl)-1H-imidazole;
4-(2-fluoro-6-spiro-1'-cyclopropane-1a,6-dihydro-1H-cyclopropa[a]inden-6a-yl)-1H-imidazole;
4-(3-fluoro-6-spiro-1'-cyclopropane-1a,6-dihydro-1H-cyclopropa[a]inden-6a-yl)-1H-imidazole;
4-(4-fluoro-6-spiro-1'-cyclopropane-1a,6-dihydro-1H-cyclopropa[a]inden-6a-yl)-1H-imidazole;
4-(5-fluoro-6-spiro-1'-cyclopropane-1a,6-dihydro-1H-cyclopropa[a]inden-6a-yl)-1H-imidazole;
4-(1-exo-methyl-6-spiro-1'-cyclopropane-1a,6-dihydro-1H-cyclopropa[a]inden-6a-yl)-1H-imidazole;
4-(1-exo-methyl-2-fluoro-6-spiro-1'-cyclopropane-1a,6-dihydro-1H-cyclopropa[a]inden-6a-yl)-1H-imidazole;
4-(1-exo-methyl-3-fluoro-6-spiro-1'-cyclopropane-1a,6-dihydro-1H-cyclopropa[a]inden-6a-yl)-1H-imidazole;
4-(1-exo-methyl-4-fluoro-6-spiro-1'-cyclopropane-1a,6-dihydro-1H-cyclopropa[a]inden-6a-yl)-1H-imidazole;
4-(1-exo-methyl-5-fluoro-6-spiro-1'-cyclopropane-1a,6-dihydro-1H-cyclopropa[a]inden-6a-yl)-1H-imidazole;
4-(1-endo-ethyl-6-spiro-1'-cyclopropane-1a,6-dihydro-1H-cyclopropa[a]inden-6a-yl)-1H-imidazole;
4-(la-methyl-6-spiro-1'-cyclopropane-1a,6-dihydro-1H-cyclopropa[a]inden-6a-yl)-1H-imidazole;
their addition salts and, if applicable, addition salt hydrates with pharmaceutically acceptable mineral acids or organic acids along with their tautomeric forms, the enantiomers, and the mixtures of enantiomers and the pure stereoisomers or racemic or non-racemic mixtures.

3. Preparation method of the compounds according to formula 1 according to claims 1 and 2, **characterised in that** a synthetic intermediate according to formula (1): 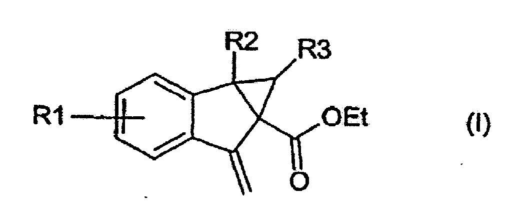 wherein R1, R2 and R3 have the same significance as in formula (1),
reacts with diiodomethane in the presence of zinc diethyl and phenol and the ester function of the compound formed is converted into a 1-H-imidazole group to obtain the compounds according to formula (1), wherein R1, R2 and R3 have the same significance as in formula (1), their addition salts and, if applicable, addition salt hydrates with pharmaceutically acceptable mineral acids or organic acids along with their tautomeric forms, the enantiomers, and the mixtures of enantiomers and the pure stereoisomers or racemic or non-racemic mixtures.

4. Compounds according to claim 1, **characterised in that** the R3 and 1H-imidazole substituents occupy syn-periplanar positions with reference to the plane defined by the cyclopropane nucleus, R1 and R2 have the same significance as in formula (1) and R3 is a methyl group (CH₃) or an ethyl group (CH₂CH₃) .

5. Compounds according to claim 1, **characterised in that** the R3 and 1H-imidazole substituents occupy anti-periplanar positions with reference to the plane defined by the cyclopropanic nucleus, R1 and R2 have the same significance as in formula (1) and R3 is a methyl group (CH₃) or an ethyl group (CH₂CH₃) .

6. Compounds according to general formula (1), according to any of claims 1, 2, 4 or 5, **characterised in that** it is selected from the levogyral enantiomer or the dextrogyral enantiomer of the compounds according to general formula (1).

7. Compounds according to any of claims 1, 2, 4, 5 and 6 as medicinal products.

8. Pharmaceutical formulations **characterised in that** they contain, as the active ingredient, at least one of the derivatives according to any of claims 1, 2, 4, 5 and 6, associated with an inert pharmaceutical substrate or other pharmaceutically acceptable vehicles or, if applicable, another medicinal product.

9. Pharmaceutical formulations according to claim 8, useful in the treatment of Alzheimer's disease or useful in the treatment of the progression of Alzheimer's disease.

10. Pharmaceutical compositions according to 8, useful in the treatment of Parkinson's disease or in the treatment of the evolution of Parkinson's disease.

## Patentansprüche

1. Verbindungen der allgemeinen Formel (1): 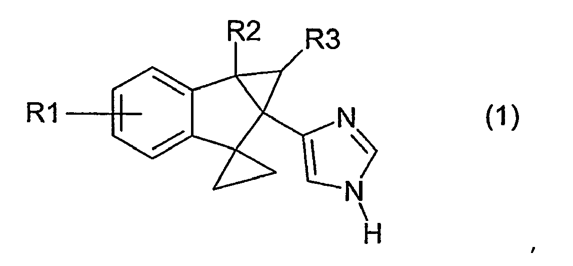 in der:
- R1 für ein Wasserstoffatom, ein Fluoratom oder eine Methoxylgruppe (OCH₃) steht, wobei der Substituent R1 an dem aromatischen Carbocyclus die 2-, 3-, 4- oder 5-Stellung belegen kann;
- R2 für ein Wasserstoffatom oder eine Methylgruppe steht;
- R3 für ein Wasserstoffatom, eine Methylgruppe oder eine Ethylgruppe steht;
deren Additionssalze und gegebenenfalls die Hydrate der Additionssalze mit den pharmazeutisch unbedenklichen anorganischen Säuren oder organischen Säuren sowie deren tautomeren Formen, den Enantiomeren und den Enantiomerengemischen und den Stereoisomeren, in racemischen Gemischen oder nicht.

2. Verbindungen nach Anspruch 1, **dadurch gekennzeichnet, dass** sie aus den folgenden Verbindungen ausgewählt sind:
4-(6-spiro-1'-Cyclopropan-1a,6-dihydro-1H-cyclopropa[a]inden-6a-yl)-1H-imidazol;
4-(2-Fluor-6-spiro-1'-cyclopropan-1a,6-dihydro-1H-cyclopropa[a]inden-6a-yl)-1H-imidazol;
4-(3-Fluor-6-spiro-1'-cyclopropan-1a,6-dihydro-1H-cyclopropa[a]inden-6a-yl)-1H-imidazol;
4-(4-Fluor-6-spiro-1'-cyclopropan-1a,6-dihydro-1H-cyclopropa[a]inden-6a-yl)-1H-imidazol;
4-(5-Fluor-6-spiro-1'-cyclopropan-1a,6-dihydro-1H-cyclopropa[a]inden-6a-yl)-1H-imidazol;
4-(1-exo-Methyl-6-spiro-1'-cyclopropan-1a,6-dihydro-1H-cyclopropa[a]inden-6a-yl)-1H-imidazol;
4-(1-exo-Methyl-2-fluor-6-spiro-1'-cyclopropan-1a,6-dihydro-1H-cyclopropa[a]inden-6a-yl)-1H-imidazol;
4-(1-exo-Methyl-3-fluor-6-spiro-1'-cyclopropan-1a,6-dihydro-1H-cyclopropa[a]inden-6a-yl)-1H-imidazol;
4-(1-exo-Methyl-4-fluor-6-spiro-1'-cyclopropan-1a,6-dihydro-1H-cyclopropa[a]inden-6a-yl)-1H-imidazol;
4-(1-exo-Methyl-5-fluor-6-spiro-1'-cyclopropan-1a,6-dihydro-1H-cyclopropa[a]inden-6a-yl)-1H-imidazol;
4-(1-endo-Ethyl-6-spiro-1'-cyclopropan-1a,6-dihydro-1H-cyclopropa[a]inden-6a-yl)-1H-imidazol;
4-(1a-Methyl-6-spiro-1'-cyclopropan-1a,6-dihydro-1H-cyclopropa[a]inden-6a-yl)-1H-imidazol;
deren Additionssalze und gegebenenfalls die Hydrate der Additionssalze mit den pharmazeutisch unbedenklichen anorganischen Säuren oder organischen Säuren sowie deren tautomeren Formen, den Enantiomeren und den Enantiomerengemischen und den Stereoisomeren, in racemischen Gemischen oder nicht.

3. Verfahren zur Herstellung von Verbindungen der Formel (1) nach den Ansprüchen 1 und 2, **dadurch gekennzeichnet, dass** eine Synthesezwischenverbindung der Formel (I): 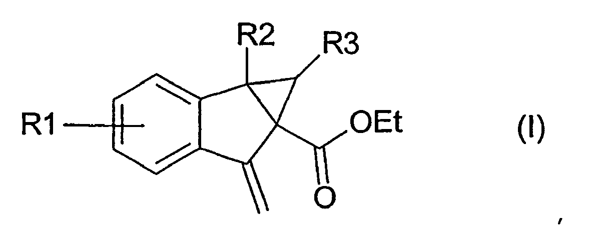 in der R1, R2 und R3 dieselbe Bedeutung wie in der Formel (1) haben,
mit Diiodmethan in Gegenwart von Zinkdiethyl und Phenol umgesetzt wird und die Esterfunktion der gebildeten Verbindung in eine 1H-Imidazolgruppe umgewandelt wird, um die Verbindungen der Formel (1) zu erhalten, in der R1, R2 und R3 dieselbe Bedeutung wie in der Formel (1) haben, deren Additionssalzen und gegebenenfalls der Hydrate der Additionssalze mit den pharmazeutisch unbedenklichen anorganischen Säuren oder organischen Säuren sowie deren tautomeren Formen, den Enantiomeren und den Enantiomerengemischen und den Stereoisomeren, in racemischen Gemischen oder nicht.

4. Verbindungen nach Anspruch 1, **dadurch gekennzeichnet, dass** die Substituenten R3 und 1H-Imidazol synperiplanare Stellungen in Bezug auf die von dem Cyclopropanring definierte Ebene belegen, R1 und R2 dieselbe Bedeutung wie in der Formel (1) haben und R3 eine Methylgruppe (CH₃) oder eine Ethylgruppe (CH₂CH₃) ist.

5. Verbindungen nach Anspruch 1, **dadurch gekennzeichnet, dass** die Substituenten R3 und 1H-Imidazol antiperiplanare Stellungen in Bezug auf die von dem Cyclopropanring definierte Ebene belegen, R1 und R2 dieselbe Bedeutung wie in der Formel (1) haben und R3 eine Methylgruppe (CH₃) oder eine Ethylgruppe (CH₂CH₃) ist.

6. Verbindungen der allgemeinen Formel (1) nach einem der Ansprüche 1, 2, 4 oder 5, **dadurch gekennzeichnet, dass** sie aus dem lävogyren Enantiomer oder dem dextrogyren Enantiomer der Verbindungen der allgemeinen Formel (1) ausgewählt sind.

7. Verbindungen nach einem der Ansprüche 1, 2, 4, 5 und 6 als Arzneimittel.

8. Pharmazeutische Zusammensetzungen, **dadurch gekennzeichnet, dass** sie als Wirkstoff mindestens eine Verbindung nach einem der Ansprüche 1, 2, 4, 5 und 6 enthalten, in Verbindung mit einem inerten pharmazeutischen Trägerstoff oder anderen pharmazeutisch unbedenklichen Exzipienten und gegebenenfalls einem weiteren Arzneimittel.

9. Pharmazeutische Zusammensetzungen nach Anspruch 8, die bei der Behandlung von Alzheimer-Krankheit verwendet werden oder bei der Behandlung der Entwicklung von Alzheimer-Krankheit verwendet werden.

10. Pharmazeutische Zusammensetzungen nach Anspruch 8, die bei der Behandlung von oder bei der Behandlung der Entwicklung von Parkinson-Krankheit verwendet werden.
